# EUROPEAN PATENT APPLICATION

(11) **EP 2 347 672 A1**
(43) Date of publication of application: **27.07.2011**
(21) Application number: 09820596.6
(22) Date of filing: 14.10.2009
(51) Int. Cl.: A45D 34/04, A45D 33/34

(54) **COSMETIC PUFF**

(30) Priority: 17.10.2008 JP 2008268963
(71) Applicant: Unicharm Corporation, Ehime 799-0111 (JP)
(72) Inventor: IKEDA, Hiroko, Kanonji-shi Kagawa 769-1602 (JP); SHIBATA, Akira, Kanonji-shi Kagawa 769-1602 (JP); OOBA, Kenji, Kanonji-shi Kagawa 769-1602 (JP); AOTO, Takashi, Kanonji-shi Kagawa 769-1602 (JP); TAKEUCHI, Naohito, Kanonji-shi Kagawa 769-1602 (JP)
(74) Representative: Peter, Julian
(86) International application number: PCT/JP2009/067775
(87) International publication number: WO 2010/044418

(57) **Abstract**

It is an object of the invention to provide an effective technique for realizing a desired moisture releasing property, in a cosmetic puff having an air-laid non-woven fabric sheet formed of pulp fibers. A cosmetic puff 100 according to this invention includes an inner layer 110 in the form of a sheet and surface layers 120 in the form of laminated sheets on the both sides of the inner layer 110. The inner layer 110 is formed by an air-laid non-woven fabric sheet containing at least pulp fibers and polyethylene terephthalate fibers.

## Description

### BACGROUND OF THE INVENTION

### Field of the Invention

The present invention relates to a technique for providing a cosmetic puff.

### Description of the Related Art

Japanese non-examined laid-open Patent Publication No. 2007-195614 discloses a cosmetic puff for use in applying skin lotion by patting. The known cosmetic puff includes an air-laid non-woven fabric sheet. In designing such cosmetic puff, it is required to provide a cosmetic puff having enhanced usability to release an appropriate amount of lotion according to the force with which a user pats the skin with the cosmetic puff impregnated with skin lotion.

### SUMMARY OF THE INVENTION

Accordingly, it is an object of the invention to provide an effective technique for releasing an appropriate amount of skin lotion according to user's patting force with respect to a cosmetic puff having an air-laid non-woven fabric sheet formed of pulp fibers.

The above-described problem can be solved by the features of the claimed invention.

The representative cosmetic puff according to this invention includes an air-laid non-woven fabric sheet manufactured by an air-laid technique. The representative cosmetic puff is preferably applied to the structure of a cosmetic puff which is used, as it is or with cosmetic liquid, powder, gel or cream contained in or on it, to pat the user's face when wearing makeup. The representative cosmetic puff particularly includes pulp fibers and hydrophobic synthetic fibers. The "hydrophobic synthetic fibers" here may include various kinds of hydrophobic synthetic fibers, and typically, one or more of polyethylene terephthalate (PET) fibers, polypropylene (PP) fibers, nylon fibers or the like can be used as hydrophobic synthetic fibers. With such a structure, by provision of the hydrophobic synthetic fibers, the cosmetic puff can provide a desired moisture releasing property, so that an appropriate amount of skin lotion can be released according to user's patting force.

Further, in a further embodiment of the cosmetic puff according to this invention, preferably, the air-laid non-woven fabric sheet contains the hydrophobic synthetic fibers at the rate of 10 to 50% by weight. With such a structure, by defining the content of the hydrophobic synthetic fibers, particularly, the rate of recovery after loaded in the wet state, the rate of one-time moisture release and the rate of fraction moisture release can be increased. The "rate of recovery after loaded" here is defined as the rate of recovery of the thickness of the specimen based on the difference of the thickness measured before and after application of a predetermined load to the wetted specimens. The "rate of one-time moisture release" here is defined as the rate of moisture release based on the amount of moisture which is released at one time from the specimen to filter paper when a predetermined load is generally applied to the wetted specimen with the filter paper stacked on top of the specimen. Further, the "rate of fraction moisture release" here is defmed as the rate of moisture release based on the amount of moisture which is finally released from the specimen to filter paper when a predetermined load is generally applied several times to the wetted specimen with the filter paper stacked on top of the specimen.

Further, in a further embodiment of the cosmetic puff according to this invention, preferably, the pulp fibers of the air-laid non-woven fabric sheet have a fiber weight per unit length of 0.20 mg/m or more. Such a pulp fiber having a relatively heavy fiber weight per unit length has an average pore size (void diameter) of 100µm or more and has a strong skeleton structure, so that the cosmetic puff having a higher rate of recovery after loaded in the wet state, a higher rate of one-time moisture release and a higher rate of fraction moisture release can be realized.

A cosmetic puff according to another embodiment of this invention includes an air-laid non-woven fabric sheet, and the air-laid non-woven fabric sheet contains pulp fibers with a fiber weight per unit length of 0.20 mg/m or more. Such a pulp fiber having a relatively heavy fiber weight per unit length has an average pore size (void diameter) of 100µm or more and has a strong skeleton structure, so that the cosmetic puff having a higher rate of recovery after loaded in the wet state, a higher rate of one-time moisture release and a higher rate of fraction moisture release can be realized.

In a further embodiment of the cosmetic puff according to this invention, preferably, the air-laid non-woven fabric sheet includes surface layers, and at least one of the surface layers is formed of rayon fibers. In this respect, either one of the surface layers of the air-laid non-woven fabric sheet may be formed of rayon fibers, or both of the surface layers of the air-laid non-woven fabric sheet may be formed of rayon fibers. With such a structure, the cosmetic puff can provide a smooth texture in use.

In a further embodiment of the cosmetic puff according to this invention, preferably, at least one of the surface layers of the air-laid non-woven fabric sheet includes moisturizer application regions to which a moisturizer is applied and moisturizer non-application regions which are regions other than the moisturizer application regions. Further, this surface layer has an area in which the moisturizer application regions and the moisturizer non-application regions are alternately arranged at least in one of the directions of the width and the length of the air-laid non-woven fabric sheet. In this respect, either one of the surface layers of the air-laid non-woven fabric sheet may include an area in which the moisturizer application regions and the moisturizer non-application regions are alternately arranged, or both of the surface layers of the air-laid non-woven fabric sheet may include an area in which the moisturizer application regions and the moisturizer non-application regions are alternately arranged.

The "moisturizer" here may preferably include diglycerin, triglycerin, ethylene glycol, propylene glycol, 1,3-butylene glycol, liquid paraffm, squalane, vaseline, olive oil, soy oil, safflower oil, camellia oil, jojoba oil, almond oil, castor oil, palm oil, mink oil, vitamin E, dimethyl silicone, silicone oil, modified silicone, sorbitol, sucrose, glucose, maltose, bees wax, hyaluronan, placental extract, aloe extract, chamomilla recutita, peach leaf extract, collagen, loofah extract, hinokitiol.

With such a structure, in the moisturizer application regions, the cosmetic puff can be provided with a moist texture and thus can provide an improved texture. The moisturizer non-application regions are kept with an apparent sheet thickness (kept bulky) without application of the moisturizer, so that it is effective in improving the appearance of the cosmetic puff (adding a quality appearance to it). Therefore, the cosmetic puff can be provided with both of an improved texture and an improved appearance.

As described above, according to this invention, in a cosmetic puff having an air-laid non-woven fabric sheet, particularly, by provision of a structure having pulp fibers and hydrophobic synthetic fibers or a structure having pulp fibers with a fiber weight per unit length of 0.20 mg/m or more, an effective technique for releasing an appropriate amount of skin lotion according to user's patting force can be provided. Other objects, features and advantages of the present invention will be readily understood after reading the following detailed description together with the accompanying drawings and the claims.

### DETAILED DESCRIPTION OF THE INVENTION

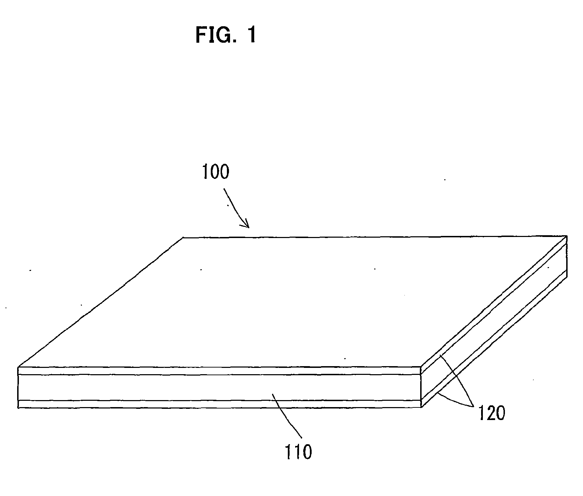

Each of the additional features and method steps disclosed above and below may be utilized separately or in conjunction with other features and method steps to provide and manufacture improved cosmetic puffs and method for using such cosmetic puffs and devices utilized therein. Representative examples of the present invention, which examples utilized many of these additional features and method steps in conjunction, will now be described in detail with reference to the drawings. This detailed description is merely intended to teach a person skilled in the art further details for practicing preferred aspects of the present teachings and is not intended to limit the scope of the invention. Only the claims define the scope of the claimed invention. Therefore, combinations of features and steps disclosed within the following detailed description may not be necessary to practice the invention in the broadest sense, and are instead taught merely to particularly describe some representative examples of the invention, which detailed description will now be given with reference to the accompanying drawings.
A representative cosmetic puff 100 as a preferred embodiment according to the invention is now described with reference to the drawings. FIG. 1 is a perspective external view showing a cosmetic puff 100 according to this embodiment.

As shown in FIG. 1, the cosmetic puff 100 according to this embodiment has a sheet-like or plate-like form as a whole and includes an inner layer 110 in the form of a sheet and surface layers 120 in the form of laminated sheets on the both sides of the inner layer 110. The cosmetic puff 100 is formed by an air-laid non-woven fabric sheet which is manufactured by a known air-laid technique. The cosmetic puff 100 is a cosmetic tool which is used, as it is or with cosmetic liquid, powder, cream or gel contained in or on it, to pat the user's face when wearing makeup. The cosmetic puff is also referred to as a "cosmetic cotton". Each of the surface layers 120 is typically formed by a non-woven fabric primarily made of at least one kind of fibers of rayon, cotton, silk, pulp or the like. The inner layer 110 contains at least pulp fibers and polyethylene terephthalate (PET) fibers. Alternatively, the inner layer 110 may be formed by an air-laid non-woven fabric sheet containing pulp fibers and not containing polyethylene terephthalate (PET) fibers, as necessary. Further, the surface layers 120 may be formed by the same fibers as the inner layer 110. The inner layer 110 and the surface layers 120 are features that correspond to the "air-laid non-woven fabric sheet" and the "surface layers", respectively, according to this invention. Further, the pulp fibers and polyethylene terephthalate fibers in the inner layer 110 are features that correspond to the "pulp fibers" and the "hydrophobic synthetic fibers", respectively, according to this invention.

In developing cosmetic puffs of this kind, particularly in order to realize release of an appropriate amount of skin lotion according to the force with which a user pats his or her skin with a cosmetic puff impregnated with skin lotion, it is required to provide the cosmetic puff having a desired moisture releasing property so that the lotion once retained in the cosmetic puff can be released by an appropriate amount under predetermined conditions. Therefore, applicants of this invention have studied on the structure and performance of cosmetic puffs of this kind. As the results of the study, they successfully found that a cosmetic puff having a desired moisture releasing property can be realized by forming the above-described inner layer 110 in the form of a non-wove fabric sheet which is formed by an air-laid non-wove fabric sheet containing pulp fibers and hydrophobic synthetic fibers.

Now, performance evaluations conducted for quantitative verification of the effects of the cosmetic puff according to the invention are explained in detail. For the performance evaluations, particularly, criterion A (rate of recovery after loaded), criterion B (rate of one-time moisture release), and criterion C (rate of fraction moisture release) were measured on each of specimens in working examples 1 to 7 and comparative examples 1 and 2 which are described below. The "rate of recovery after loaded" here is defmed as the rate of recovery of the thickness of the specimen based on the difference of the thickness measured before and after application of a predetermined load to the wetted specimens. The "rate of one-time moisture release" here is defined as the rate of moisture release based on the amount of moisture which is released at one time from the specimen to filter paper when a predetermined load is generally applied to the wetted specimen with the filter paper stacked on top of the specimen. Further, the "rate of fraction moisture release" here is defined as the rate of moisture release based on the amount of moisture which is finally released from the specimen to filter paper when a predetermined load is generally applied several times to the wetted specimen with the filter paper stacked on top of the specimen.

### (Specimen of working example 1)

As for the specimen of the working example 1, a non-woven fabric (weight: approx. 60 g/m²) made of pulp fibers having a medium fiber diameter (fiber length: 2.58 mm, fiber weight: 0.20 mg/m) is used as the air-laid non-wove fabric sheet in the form of the inner layer 110, and rayon fibers (1.7dot × 29 mm) are used as the non-wove fabric in the form of the surface layers 120. The ratio (weight percentage) of mixture of the pulp fibers and the rayon fibers is set to 93 : 7. Further, the mixture ratio of a binder or typically styrene-butadiene rubber is set to 12.5% by weight.

### (Specimen of working example 2)

As for the specimen of the working example 2, a non-woven fabric (weight: approx. 60 g/m²) made of pulp fibers having a medium fiber diameter (fiber length: 2.58 mm, fiber weight: 0.20 mg/m) and polyethylene terephthalate fibers (2.2dt × 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 83 : 10 : 7. Further, the mixture ratio of the same binder as in the working example 1 is set to 12.5% by weight.

### (Specimen of working example 3)

As for the specimen of the working example 3, a non-woven fabric (weight: approx. 60 g/m²) made of pulp fibers having a medium fiber diameter (fiber length: 2.58 mm, fiber weight: 0.20 mg/m) and polyethylene terephthalate fibers (2.2dt × 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 73 : 20 : 7. Further, the mixture ratio of the same binder as in the working example 1 is set to 12.5% by weight.

### (Specimen of working example 4)

As for the specimen of the working example 4, a non-woven fabric (weight: approx. 60 g/m²) made of pulp fibers having a medium fiber diameter (fiber length: 2.58 mm, fiber weight: 0.20 mg/m) and polyethylene terephthalate fibers (2.2dt × 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 63 : 30 : 7. Further, the mixture ratio of the same binder as in the working example 1 is set to 12.5% by weight.

### (Specimen of working example 5)

As for the specimen of the working example 5, a non-woven fabric (weight: approx. 60 g/m²) made of pulp fibers having a medium fiber diameter (fiber length: 2.58 mm, fiber weight: 0.20 mg/m) and polyethylene terephthalate fibers (2.2dt × 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 63 : 30 : 7. Further, the mixture ratio of the same binder as in the working example 1 is set to 7.5% by weight.

### (Specimen of working example 6)

As for the specimen of the working example 6, a non-woven fabric (weight: approx. 60 g/m²) made of pulp fibers having a medium fiber diameter (fiber length: 2.58 mm, fiber weight: 0.20 mg/m) and polyethylene terephthalate fibers (2.2dt × 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 63 : 30 : 7. Further, the mixture ratio of the same binder as in the working example 1 is set to 15.0% by weight.

### (Specimen of working example 7)

As for the specimen of the working example 7, a non-woven fabric (weight: approx. 60 g/m²) made of pulp fibers having a relatively large fiber diameter (fiber length: 2.77 mm, fiber weight: 0.24 mg/m) and polyethylene terephthalate fibers (2.2dt x 5 mm) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers, the polyethylene terephthalate fibers and the rayon fibers is set to 63 : 30 : 7. Further, the mixture ratio of the same binder as in the working example 1 is set to 12.5% by weight.

### (Specimen of comparative example 1)

As for the specimen of comparative example 1, a non-woven fabric (weight: approx. 60 g/m²) made of pulp fibers having a relatively small fiber diameter (fiber length: 2.42 mm, fiber weight: 0.17 mg/m) is used as the air-laid non-wove fabric sheet in the form of the inner layer 110. Further, the mixture ratio of the same binder as in the working example 1 is set to 10.0% by weight.

### (Specimen of comparative example 2)

As for the specimen of comparative example 2, a non-woven fabric (weight: approx. 60 g/m²) made of pulp fibers having a relatively small fiber diameter (fiber length: 2.42 mm, fiber weight: 0.17 mg/m) is used as the air-laid non-wove fabric sheet in the form of the inner layer 110, and rayon fibers (1.7dt × 29 mm) are used as the non-wove fabric in the form of the surface layers 120. The ratio (weight percentage) of mixture of the pulp fibers and the rayon fibers is set to 93 : 7. Further, the mixture ratio of the same binder as in the working example 1 is set to 12.5% by weight.

Further, measurements and evaluations on each of criterion A (rate of recovery after loaded), criterion B (rate of one-time moisture release), criterion C (rate of fraction moisture release) have been made as follows:

### (Rate of recovery after loaded)

For measurements of the rate of recovery after loaded, a portable thickness gage (CR-10A of Daiei Kagaku Seiki Mfg. Co., Ltd.) is used to measure the thickness of each of the wetted specimens while applying a first pressure (1.96 kPa (20 gf/cm²)) or a second pressure (3.92 kPa (40 gf/cm²)) by a pressure-foot (1 cm² (ϕ 11.28 mm)). The rate of recovery after loaded is obtained by the equation of (Dc - Db )/Db × 100.

### (Rate of one-time moisture release)

For measurements of the rate of one-time moisture release, first, filter paper having dimensions of 50 × 50 mm is laid on top of each specimen having dimensions of 50 × 50 mm and wetted (impregnated with 2 ml of test liquid). Thereafter, a predetermined pressure (17.5 kgf/ cm²) is applied thereto and the amount of moisture released from each specimen to the filter case is calculated. Specifically, a weight Wa of each wetted specimen and a weight Wb of ten pieces of filter paper are measured in advance, and then the ten pieces of filter paper (of the weight Wb) are laid on top of each wetted specimen. Subsequently, the above-described predetermined pressure is applied to the filter paper from above for 15 seconds and thereafter a weight Wc of the ten pieces of filter paper is measured. Then, the rate of one-time moisture release (%) is obtained by the equation of (Wc - Wb)/Wa × 100.

### (Rate of fraction moisture release)

For measurements of the rate of traction moisture release, first, filter paper having dimensions of 50 × 50 mm is laid on top of each specimen having dimensions of 50 × 50 mm and wetted (impregnated with 2 ml of test liquid). Thereafter, a predetermined pressure (34.6 kgf/ cm²) is applied thereto in three steps and the amount of moisture released from each specimen to the filter case is calculated after each application of pressure. Specifically, in the first step, a weight Wd of each wetted specimen and a weight We of ten pieces of filter paper are measured in advance, and then the ten pieces of filter paper (of the weight We) are laid on top of each wetted specimen. Subsequently, the above-described predetermined pressure is applied to the filter paper from above for 5 seconds and thereafter a weight Wf of the ten pieces of filter paper is measured. Then, the rate of the first moisture release (%) is obtained by the equation of (Wf - We)/Wd × 100.

Subsequently, in the second step, a weight Wg of ten new pieces of filter paper is measured, and then the ten pieces of filter paper (of the weight Wg) are laid on top of each wetted specimen obtained in the first step. Subsequently, the above-described predetermined pressure is applied to the filter paper from above for 5 seconds and thereafter a weight Wh of the ten pieces of filter paper is measured. Subsequently, in the third step, a weight Wi of another ten new pieces of filter paper is measured, and then the ten pieces of filter paper (of the weight Wi) are laid on top of each wetted specimen obtained in the second step. Subsequently, the above-described predetermined pressure is applied to the filter paper from above for 5 seconds and thereafter a weight Wj of the ten pieces of filter paper is measured. Then, the rate of the second moisture release (%) is obtained by the equation of (Wh - Wg)/Wd × 100.

Subsequently, in the third step, a weight Wg of ten new pieces of filter paper is measured, and then the ten pieces of filter paper (of the weight Wg) are laid on top of each wetted specimen obtained in the first step. Subsequently, the above-described predetermined pressure is applied to the filter paper from above for 5 seconds and thereafter a weight Wh of the ten pieces of filter paper is measured. Subsequently, in the third step, a weight Wi of another ten new pieces of filter paper is measured, and then the ten pieces of filter paper (of the weight Wi) are laid on top of each wetted specimen obtained in the second step. Subsequently, the above-described predetermined pressure is applied to the filter paper from above for 5 seconds and thereafter a weight Wj of the ten pieces of filter paper is measured. Then, the rate of the third moisture release (%) is obtained by the equation of (Wj - Wi)/Wd × 100.

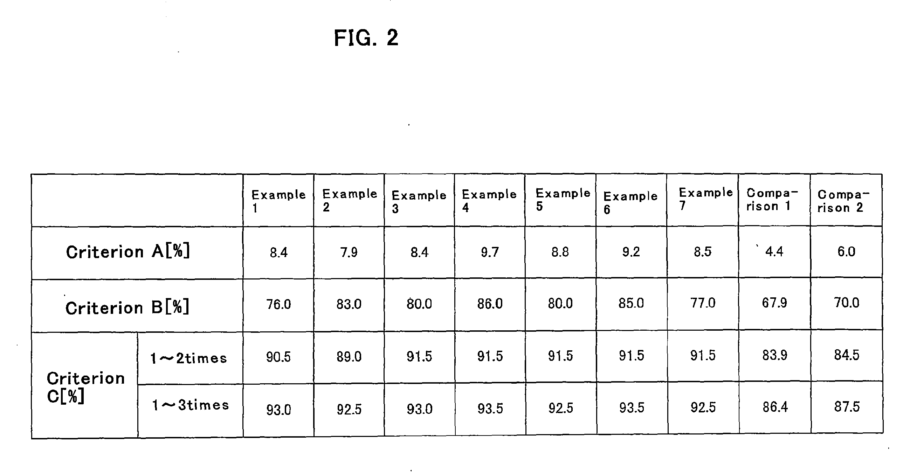

FIG. 2 shows results of evaluation of each specimen in the working examples 1 to 7 and the comparative examples 1 and 2 in this embodiment on criteria A, B, C. Further, in this embodiment, the fiber length and the fiber weight of the pulp fibers are the "average fiber length" and the fiber weight (also referred to as the "fiber roughness") per unit length, respectively. From the evaluation results shown in FIG. 2, evaluations are made as follows:

### (Evaluation results on criterion A)

As for the criterion A, the rate of recovery after loaded of every specimen in the working examples 1 to 7 exceeds 7%, and it is thus verified that the specimens in the working examples 1 to 7 have higher performance of recovery after loaded, compared with the specimens of the comparative examples 1 and 2 in which the rate of recovery after loaded is below 7%. Therefore, when, for example, a cosmetic puff impregnated with skin lotion is used for patting, a sufficient amount of release of skin lotion can be provided. Further, due to the higher performance of releasing skin lotion, such a cosmetic puff is economical without wasting skin lotion

### (Evaluation results on criterion B)

As for the criterion B, the rate of one-time moisture release of every specimen in the working examples 1 to 7 exceeds 75%, and it is thus verified that the specimens in the working examples 1 to 7 have higher performance of one-time moisture release (at one time or in one step), compared with the specimens of the comparative examples 1 and 2 in which the rate of one-time moisture release is below 75%. Therefore, when, for example, a cosmetic puff impregnated with skin lotion is used for patting, a sufficient amount of release of skin lotion can be provided even by lightly patting the skin. In other words, it is not necessary to pat the skin with a strong force which may put a strain on the skin.

### (Evaluation results on criterion C)

As for the criterion C, in the working examples 1 to 7, the rate of (first to second) moisture release of every specimen exceeds 85%, and the rate of (first to third) moisture release of every specimen exceeds 90%. It is thus verified that the specimens in the working examples 1 to 7 have higher performance of fraction moisture release (in several steps), compared with the specimens of the comparative examples 1 and 2 in which the rate of (first to second) moisture release is below 85% and the rate of (first to third) moisture release is below 90%. Therefore, when, for example, a cosmetic puff impregnated with skin lotion is used for patting several times (in several steps), a sufficient amount of release of skin lotion can be continuously provided. Application of skin lotion to the skin by patting in several steps is effective for increasing moisture in the skin.

As described above, by using an air-laid non-woven fabric sheet corresponding to any one of the specimens of the working examples 1 to 7, a cosmetic puff having enhanced usability can be provided by designing it to release an appropriate amount of skin lotion according to the force with which a user pats the skin with the cosmetic puff impregnated with skin lotion.
Specifically, each of the air-laid non-woven fabric sheets corresponding to the specimens of the working examples 1 to 7 contains pulp fibers with a fiber weight per unit length of 0.20 mg/m or more. Such a pulp fiber has an average pore size (void diameter) of 100µm or more and has a strong skeleton structure, so that the rate of recovery after loaded in the wet state, the rate of one-time moisture release and the rate of fraction moisture release can be increased. Thus, such a sheet is effective for gradually discharging the skin lotion retained in space between the fibers, to the surface layer. Further, each of the air-laid non-woven fabric sheets corresponding to the specimens of the working examples 2 to 6 contains 10 to 50% of polyethylene terephthalate fibers by weight. Therefore, in this case, the rate of recovery after loaded in the wet state, the rate of one-time moisture release and the rate of fraction moisture release can be further increased, so that the effect of gradually discharging the skin lotion retained in space between the fibers, to the surface layer can be further enhanced.
Further, each of the air-laid non-woven fabric sheets corresponding to the specimens of the working examples 1 to 7 contains rayon fibers in areas corresponding to the surface layers 120, so that the cosmetic puff provide a smooth texture in use. Either one of the two surface layers 120 may be formed by the rayon fibers, as necessary.

Further, the mixture ratio of a binder contained in the air-laid non-woven fabric sheet is preferably set to 5 to 15% by weight. If the mixture ratio of the binder is decreased to below 5% by weight, disadvantageously, the rate of recovery after loaded in the wet state is decreased, and the amount of first moisture release is increased. Further, if the mixture ratio of the binder exceeds 15% by weight, disadvantageously, the material is hardened and rendered unsuitable for a cosmetic puff.

Further, the content of polyethylene terephthalate fibers in the air-laid non-woven fabric sheet is preferably set to 10 to 50% by weight. The polyethylene terephthalate fibers are hydrophobic, and if the content of polyethylene terephthalate fibers exceeds 50% by weight, disadvantageously, most of the skin lotion absorbed therein is immediately released by first patting, and an increase in the amount of polyethylene terephthalate fibers leads to cost increase. Further, alternatively or in addition to the polyethylene terephthalate fibers, other hydrophobic synthetic fibers may be used. In this case, polypropylene (PP) fibers, nylon fibers or the like can be used as the other hydrophobic synthetic fibers.

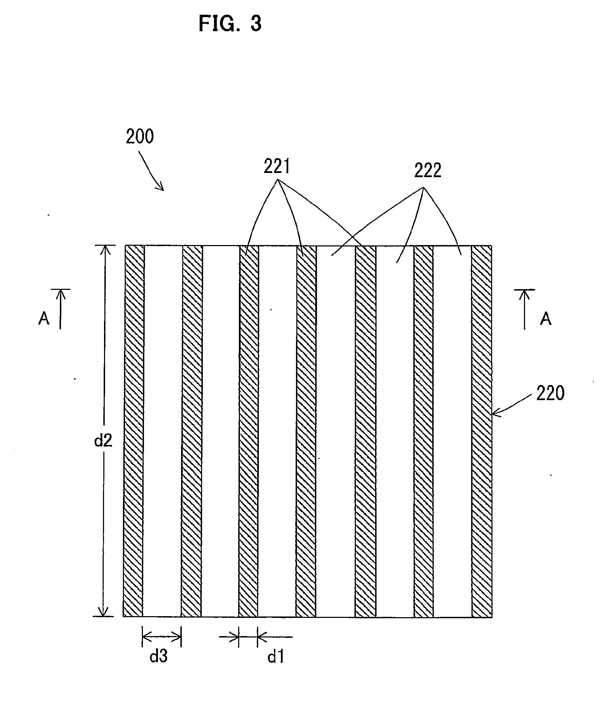

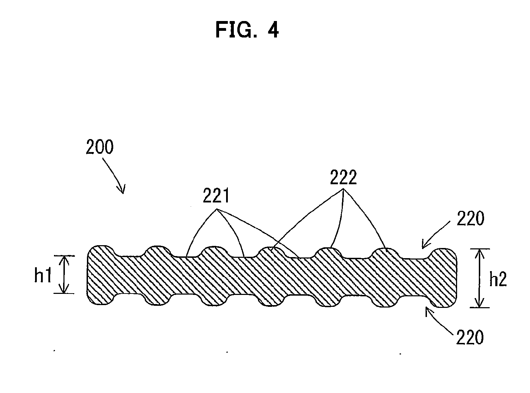

In the cosmetic puff 100 having the above-described structure, preferably, a moisturizer is applied to the surface layers 120 in order to provide a moist texture. Now, a cosmetic puff 200 with a moisturizer applied thereto is explained with reference to FIGS. 3 and 4. FIG. 3 is a plan view of the cosmetic puff 200 according to this embodiment, and FIG. 4 is a sectional view of the cosmetic puff 200 which is taken along line A-A in FIG. 3.

As shown in FIG. 3, the cosmetic puff 200 as a representative embodiment according to the invention has surface layers 220 each including moisturizer application regions 221 to which a moisturizer is applied and moisturizer non-application regions 222 or regions other than the moisturizer application regions 221. The cosmetic puff 200 has an area in which the moisturizer application regions 221 and the moisturizer non-application regions 222 are alternately arranged in the direction of the width of the cosmetic puff 200 (which direction is set to coincide with the horizontal direction in FIG. 3). Specifically, each of the moisturizer application regions 221 is defined as an elongated region having an application width d1 in the horizontal direction and an application width d2 in the vertical direction. The moisturizer application regions 221 are arranged with pitches d3 in the horizontal direction. Further, each of the moisturizer non-application regions 222 is defmed as a region between adjacent moisturizer application regions 221. As shown in FIG. 4, due to application of the moisturizer, a sheet thickness h1 of the moisturizer application region 221 is rendered smaller than a sheet thickness h2 of the moisturizer non-application region 222. By such arrangement of the moisturizer application regions 221 shown in FIG. 3, a so-called "stripe pattern" is formed. The moisturizer application regions 221 and the moisturizer non-application regions 222 here are features that correspond to the "moisturizer application regions" and the "moisturizer non-application regions", respectively, according to this invention.

As the moisturizer to be applied to the moisturizer application regions 221, typically, glycerin can be used. This moisturizer has a softening property as well as the moisturizing property, so that it is also referred to as a softener. As the moisturizer other than glycerin, at least one of the following materials can be used: diglycerin, triglycerin, ethylene glycol, propylene glycol, 1,3-butylene glycol, liquid paraffin, squalane, vaseline, olive oil, soy oil, safflower oil, camellia oil, jojoba oil, almond oil, castor oil, palm oil, mink oil, vitamin E, dimethyl silicone, silicone oil, modified silicone, sorbitol, sucrose, glucose, maltose, bees wax, hyaluronan, placental extract, aloe extract, chamomilla recutita, peach leaf extract, collagen, loofah extract, hinokitiol. Further, polyhydric alcohol or surface active agent may also be added as an auxiliary to the moisturizer.

With such a structure, in the moisturizer application regions 221, the cosmetic puff can be provided with a moist texture and thus can provide an improved texture. The moisturizer non-application regions 222 are kept with an apparent sheet thickness (kept bulky) without application of the moisturizer as shown in FIG. 4, so that it is effective in improving the appearance of the cosmetic puff (adding a quality appearance to it). Therefore, the cosmetic puff can be provided with both of an improved texture and an improved appearance.

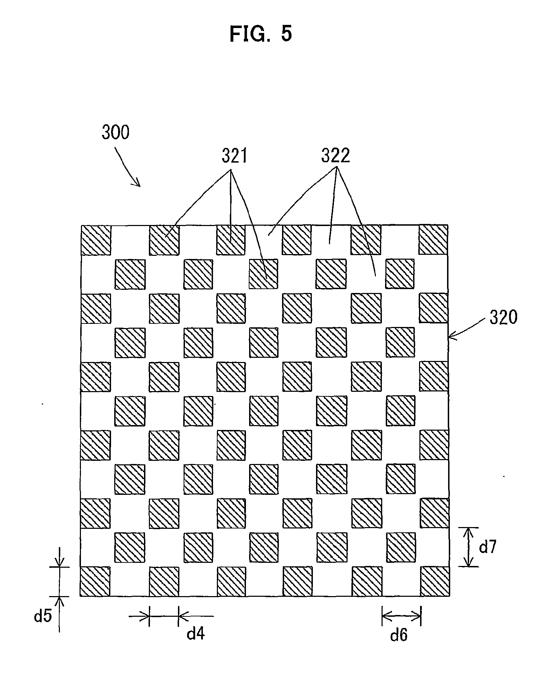

To this end, preferably, the moisturizer application regions 221 and the moisturizer non-application regions 222 are evenly arranged over the entire surface layers 220. Therefore, instead of the arrangement of the moisturizer application regions 221 and the moisturizer non-application regions 222 in the cosmetic puff 200 as shown in FIG. 3, for example, an arrangemen shown in FIG. 5 may be used. FIG. 5 is a plan view of a cosmetic puff 300 according to another embodiment of the present invention.

As shown in FIG. 5, the cosmetic puff 300 as a representative embodiment of the invention has surface layers 320 each including moisturizer application regions 321 to which a moisturizer is applied and moisturizer non-application regions 322 or regions other than the moisturizer application regions 321. The cosmetic puff 300 has an area in which the moisturizer application regions 321 and the moisturizer non-application regions 322 are alternately arranged in the directions of the width and the length of the cosmetic puff 200 (wherein the direction of the width is set to coincide with the horizontal direction in FIG. 5). Specifically, each of the moisturizer application regions 321 is defined as a rectangular region (typically a square region) having an application width d4 in the horizontal direction and an application width d5 in the vertical direction. The moisturizer application regions 321 are arranged with pitches d6 in the horizontal direction and with pitches d7 in the vertical direction. Further, each of the moisturizer non-application regions 322 is defined as a region between adjacent moisturizer application regions 321. Like in the case shown in FIG. 4, the sheet thickness of the moisturizer application region 321 is rendered smaller than the sheet thickness of the moisturizer non-application region 322. By such arrangement of the moisturizer application regions 321 shown in FIG. 5, a so-called "lattice pattern" or "checkered pattern" is formed. The moisturizer application regions 321 and the moisturizer non-application regions 322 here are features that correspond to the "moisturizer application regions" and the "moisturizer non-application regions", respectively, according to this invention.

As described above with reference to FIGS. 3 to 5, in this embodiment, the moisturizer application regions and the moisturizer non-application regions are preferably provided in the surface layers of the cosmetic puff. Applicants of this invention further found successfully that improvements in texture and appearance can both be realized at a higher level by defining the shape, dimensions, pitches or the like of the moisturizer application regions.

Specifically, applicants conducted quantitative evaluations of the texture and the appearance of the moisturizer application regions 221 shown in FIG. 3 and the moisturizer application regions 321 shown in FIG. 5 in varying dimensions and pitches. For the quantitative evaluations, particularly, criterion D (dry thickness) and criterion E (flexural property) were measured on each of specimens in working examples 11 to 16 and comparative examples 11 to 14.

### (Specimen of working example 11)

As for the specimen of the working example 11, the specimen of the working example 1 is used as a base, and a moisturizer (glycerin: water = 70% by weight: 30% by weight) is applied to the both sides of the specimen at the rate of 20% by weight. Further, a plurality of the moisturizer application regions 221 shown in FIG. 3 are arranged to extend in the vertical direction, having the application width d1 of 2.0 mm and the pitches d3 of 5.0 mm.

### (Specimen of working example 12)

As for the specimen of the working example 12, the specimen of the working example 1 is used as a base, and a moisturizer (glycerin : water = 70% by weight: 30% by weight) is applied to the both sides of the specimen at the rate of 20% by weight. Further, a plurality of the moisturizer application regions 221 shown in FIG. 3 are arranged to extend in the vertical direction, having the application width d1 of 2.0 mm and the pitches d3 of 2.0 mm.

### (Specimen of working example 13)

As for the specimen of the working example 13, the specimen of the working example 1 is used as a base, and a moisturizer (glycerin: water = 70% by weight: 30% by weight) is applied to the both sides of the specimen at the rate of 20% by weight. Further, a plurality of the moisturizer application regions 221 shown in FIG. 3 are arranged to extend in the vertical direction, having the application width d1 of 1.0 mm and the pitches d3 of 5.0 mm.

### (Specimen of working example 14)

As for the specimen of the working example 14, the specimen of the working example 1 is used as a base, and a moisturizer (glycerin : water = 70% by weight: 30% by weight) is applied to the both sides of the specimen at the rate of 20% by weight. Further, a plurality of the moisturizer application regions 221 shown in FIG. 3 are arranged to extend in the vertical direction, having the application width d1 of 1.0 mm and the pitches d3 of 2.0 mm.

### (Specimen of working example 15)

As for the specimen of the working example 15, the specimen of the working example 1 is used as a base, and a moisturizer (glycerin : water = 70% by weight : 30% by weight) is applied to the both sides of the specimen at the rate of 20% by weight. Further, a plurality of the moisturizer application regions 321 shown in FIG. 5 are arranged, having the application widths d1, d2 (d1 = d2) of 2.0 mm, the horizontal pitches d6 of 4.0 mm and the vertical pitches d7 of 6.0 mm.

### (Specimen of working example 16)

As for the specimen of the working example 16, the specimen of the working example 1 is used as a base, and a moisturizer (glycerin: water = 70% by weight: 30% by weight) is applied to the both sides of the specimen at the rate of 20% by weight. Further, a plurality of the moisturizer application regions 221 shown in FIG. 3 are arranged to extend in the horizontal direction, having the application width d1 of 2.0 mm and the pitches d3 of 5.0 mm.

### (Specimen of comparative example 11)

As for the specimen of the comparative example 11, the specimen of the working example 1 is used without application of a moisturizer and as a basis of evaluation, i.e. "blank" of the other specimens.

### (Specimen of comparative example 12)

As for the specimen of the comparative example 12, the specimen of the working example 1 is used as a base, and a moisturizer (glycerin: water = 70% by weight: 30% by weight) is applied all over the both sides of the specimen.

### (Specimen of comparative example 13)

As for the specimen of the comparative example 13, the specimen of the working example 1 is used as a base, and a moisturizer (glycerin: water = 70% by weight: 30% by weight) is applied to the both sides of the specimen at the rate of 20% by weight. Further, a plurality of the moisturizer application regions 221 shown in FIG. 3 are arranged to extend in the vertical direction, having the application width d1 of 2.0 mm and the pitches d3 of 1.0 mm.

### (Specimen of comparative example 14)

As for the specimen of the comparative example 14, the specimen of the working example 1 is used as a base, and a moisturizer (glycerin: water = 70% by weight: 30% by weight) is applied to the both sides of the specimen at the rate of 20% by weight. Further, a plurality of the moisturizer application regions 321 shown in FIG. 5 are arranged, having the application widths d4, d5 (d4 = d5) of 2.0 mm, the horizontal pitches d6 of 3.0 mm and the vertical pitches d7 of 3.0 mm.

For evaluations on the criterion D (dry thickness), the thickness of each dry specimen (which corresponds to the sheet thickness h2 in FIG. 4) is measured by using a portable thickness gage (CR-10A of Daiei Kagaku Seiki Mfg. Co., Ltd.). For evaluations on the criterion E (flexural property), a value B (gf·cm²/cm) of the flexural property is obtained by using a flexural property measuring equipment (KES-FB2 AUTO-A of Kato Tech Co., Ltd.).

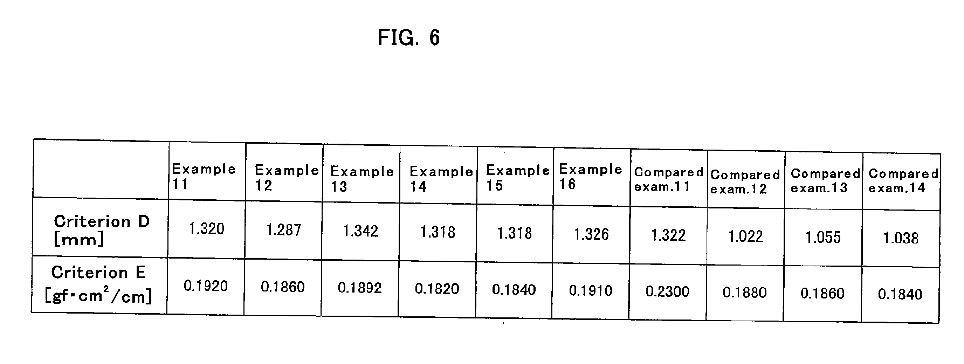

FIG. 6 shows results of evaluation of each specimen in the working examples 11 to 16 and the comparative examples 11 to 14 in this embodiment on criteria D, E. From the evaluation results shown in FIG. 6, evaluations are made as follows:

### (Evaluation results on criterion D)

As for the criterion D, the dry thickness of every specimen of the working examples 11 to 16 exceeds 1.2 mm, and it is thus verified that the specimens in the working examples 11 to 16 are kept bulky, compared with the specimens of the comparative examples 12 to 14 in which the dry thickness is far below the initial thickness (1.322 mm) of the comparative example 11. Therefore, the cosmetic puff can be improved in appearance (or provide a quality appearance).

### (Evaluation results on criterion E)

As for the criterion E, the value B (gf·cm²/cm) of the flexural property of every specimen of the working examples 11 to 16 and the comparative examples 12 to 14 is far below the value B (0.2300 gf·cm²/cm) of the comparative example 11. It is thus verified that improvement in texture is realized by application of a moisturizer.

From the above-described evaluation results on the criteria D and E, it is quantitatively verified that improvements in texture and appearance can both be realized at a higher level by forming the cosmetic puff of materials corresponding to the specimens of the working examples 11 to 16.
The specimens of the comparative examples 12 to 14 each have relatively small pitches, compared with the specimens of the working examples 11 to 16. Therefore, the pitches between adjacent moisturizer application regions are kept unchanged only shortly after application of the moisturizer, but the moisturizer may spread with the passage of time and reduce the pitches finally to almost zero (almost in the state of overall application). As a result, the dry thickness may be reduced. Therefore, if the initial pitches between adjacent moisturizer application regions can be kept unchanged even during use of the product by the user, the pitches and the application width of the moisturizer are not limited to the settings defined in the working examples 11 to 16, but can be appropriately set as necessary.

### BRIEF DESCRIPTION OF THE DRAWINGS

FIG. 1 is a perspective external view showing a cosmetic puff 100 according to an embodiment of the present invention.
FIG. 2 shows results of evaluation of each specimen in working examples 1 to 7 and comparative examples 1 and 2 on criteria A, B, C.
FIG. 3 is a plan view of a cosmetic puff 200 according to another embodiment of the present invention.
FIG. 4 is a sectional view of the cosmetic puff 200 which is taken along line A-A in FIG. 3.
FIG. 5 is a plan view of a cosmetic puff 300 according to another embodiment of the present invention.
FIG. 6 shows results of evaluation of each specimen in working examples 11 to 16 and comparative examples 11 to 14 on criteria D and E.

### Description of Numerals

100, 200, 300 cosmetic puff
110 inner layer
120, 220, 320 surface layer
221, 321 moisturizer application region
222, 322 moisturizer non-application region

## Claims

1. A cosmetic puff comprising an air-laid non-woven fabric sheet,
**characterized in that** the air-laid non-woven fabric sheet includes pulp fibers and hydrophobic synthetic fibers.

2. The cosmetic puff as defined in claim 1, wherein the air-laid non-woven fabric sheet includes the hydrophobic synthetic fibers at the rate of 10 to 50% by weight.

3. The cosmetic puff as defined in claim 1 or 2, wherein the pulp fibers of the air-laid non-woven fabric sheet have a fiber weight per unit length of 0.20 mg/m or more.

4. A cosmetic puff comprising an air-laid non-woven fabric sheet,
**characterized in that** the air-laid non-woven fabric sheet contains pulp fibers with a fiber weight per unit length of 0.20 mg/m or more.

5. The cosmetic puff as defined in any one of claims 1 to 4, wherein the air-laid non-woven fabric sheet includes surface layers, at least one of the surface layers being formed of rayon fibers.

6. The cosmetic puff as defined in any one of claims 1 to 5, wherein at least one of the surface layers of the air-laid non-woven fabric sheet includes moisturizer application regions to which a moisturizer is applied and moisturizer non-application regions which are regions other than the moisturizer application regions, and wherein said surface layer has an area in which the moisturizer application regions and the moisturizer non-application regions are alternately arranged at least in one of the directions of the width and the length of the air-laid non-woven fabric sheet.
